# EUROPÄISCHE PATENTSCHRIFT

(11) **EP 3 598 159 B1**
(45) Veröffentlichungstag und Bekanntmachung des Hinweises auf die Patenterteilung: **01.09.2021**
(21) Anmeldenummer: 18183851.7
(22) Anmeldetag: 17.07.2018
(51) Int. Cl.: G01R 33/483, G01R 33/561

(54) **VERFAHREN ZUR AUFNAHME EINES MAGNETRESONANZDATENSATZES, MAGNETRESONANZEINRICHTUNG, COMPUTERPROGRAMM UND ELEKTRONISCH LESBARER DATENTRÄGER**
METHOD FOR RECORDING A MAGNETIC RESONANCE DATA RECORD, MAGNETIC RESONANCE DEVICE, COMPUTER PROGRAM AND ELECTRONICALLY READABLE DATA CARRIER
PROCÉDÉ D'ENREGISTREMENT D'UN ENSEMBLE DE DONNÉES DE RÉSONANCE MAGNÉTIQUE, DISPOSITIF DE RÉSONANCE MAGNÉTIQUE, PROGRAMME INFORMATIQUE ET SUPPORT D'INFORMATIONS LISIBLE ÉLECTRONIQUEMENT

(43) Veröffentlichungstag der Anmeldung: 22.01.2020
(73) Patentinhaber: Siemens Healthcare GmbH, 91052 Erlangen (DE)
(72) Erfinder: Zeller, Mario, 91054 Erlangen (DE); Carinci, Flavio, 91052 Erlangen (DE); Paul, Dominik, 91088 Bubenreuth (DE)

(56) Entgegenhaltungen:
- DE-A1-102016 217 863
- MARKUS BARTH ET AL: "Simultaneous multislice (SMS) imaging techniques : SMS Imaging", MAGNETIC RESONANCE IN MEDICINE, Bd. 75, Nr. 1, 26. August 2015 (2015-08-26), Seiten 63-81, XP055408927, US ISSN: 0740-3194, DOI: 10.1002/mrm.25897
- YUN S.D. ET AL.: "Accelerated multiplexed-EPI with PSF-based distortion correction at 9.4T", PROCEEDINGS OF THE INTERNATIONAL SOCIETY FOR MAGNETIC RESONANCE IN MEDICINE, JOINT ANNUAL MEETING ISMRM-ESMRMB, Nr. 1410, 28. April 2014 (2014-04-28), Seite 1410, XP040662479, Milan, Italy
- KAWIN SETSOMPOP ET AL: "Blipped-controlled aliasing in parallel imaging for simultaneous multislice echo planar imaging with reduced g-factor penalty", MAGNETIC RESONANCE IN MEDICINE, Bd. 67, Nr. 5, 19. August 2011 (2011-08-19), Seiten 1210-1224, XP055027257, ISSN: 0740-3194, DOI: 10.1002/mrm.23097

## Beschreibung

Die Erfindung betrifft ein Verfahren zur Aufnahme eines Magnetresonanzbilddatensatzes eines Untersuchungsbereichs eines Patienten mit einer Magnetresonanzeinrichtung, wobei zur Aufnahme von Magnetresonanzdaten eine Mehrschicht-Bildgebungstechnik bei gleichzeitig zumindest teilweiser Unterabtastung in der Schichtebene angewandt wird, wobei Magnetresonanzdaten aus mehreren angeregten Schichten gleichzeitig ausgelesen und durch einen Schichttrennungsalgorithmus, der mittels in einem getrennten Referenzscan aufgenommenen Referenzdaten kalibriert ist, den gleichzeitig ausgelesenen Schichten zugeordnet werden, wonach ein die Unterabtastung in der Schichtebene kompensierender Unterabtastungsalgorithmus auf die unterabgetasteten Magnetresonanzdaten der einzelnen Schichten angewandt wird. Daneben betrifft die Erfindung eine Magnetresonanzeinrichtung, ein Computerprogramm und einen elektronisch lesbaren Datenträger.

Die Magnetresonanzbildgebung ist eine inzwischen etablierte Modalität zur Aufnahme von medizinischen Bilddaten in Untersuchungsbereichen eines Patienten. Nachdem die Dauer von Magnetresonanzuntersuchungen eines der wesentlichen Probleme der Magnetresonanzbildgebung darstellt, wurde eine Vielzahl beschleunigter Bildgebungstechniken erforscht und zur Anwendung gebracht. Hierzu zählt zum einen die Unterabtastung in der Schichtebene selbst, wobei fehlende k-Raumdaten durch entsprechende Unterabtastungsalgorithmen berechnet werden können, beispielsweise nach dem Inplane-GRAPPA-Verfahren. Ein weiterer Zugang zur beschleunigten Bildgebung ist die sogenannte simultane Mehrschichtbildgebung (Simultaneous MultiSlice - SMS). Hierbei werden durch ein gemeinsames Anregungsmodul mehrere Schichten innerhalb einer Repetition angeregt und die Magnetresonanzsignale aus diesen Schichten werden gleichzeitig vermessen. Anschließend werden Magnetresonanzdaten mittels eines Schichttrennungsalgorithmus, beispielsweise eines Schicht-GRAPPA-Algorithmus, den einzelnen, gleichzeitig aufgenommenen Schichten zugeordnet. Bekannte simultane Mehrschicht-Bildgebungstechniken umfassen beispielsweise die sogenannte Hadamard-Codierung, Verfahren mit simultaner Echo-Refokussierung, Verfahren mit Breitband-Datenaufnahme oder auch Verfahren, die eine parallele Bildgebung in Schichtrichtung einsetzen.

Eine kürzlich eingeführte Mehrschicht-Bildgebungstechnik ist die sogenannte blipped CAIPI-Technik (CAIPI - Controlled Aliasing In Parallel Imaging), die durch Setsompop et al. im Artikel "Blipped-Controlled Aliasing in Parallel Imaging for Simultaneous Multislice Echo Planar Imaging With Reduced g-Factor Penalty", Magnetic Resonance in Medicine 67, 2012, Seiten 1210 - 1224, beschrieben wird. Um mehrere Schichten gleichzeitig anzuregen, wird ein sogenannter Multiband-Puls eingesetzt, bei dem die Pulsformen für alle Bänder addiert werden, so dass ein basisbandmodulierter Multiband-Puls entsteht. Für jede angeregte Schicht wird eine lineare Phasenrampe im k-Raum in der Schichtrichtung hinzugefügt. Diese lineare Phase erzeugt eine Schichtverschiebung im Bildraum.

Um die sogenannte g-Faktor-Strafe zu reduzieren, werden Bildverschiebungen zwischen den Schichten während des Auslesemoduls induziert, indem entweder Gradienten-Blips in Schichtrichtung ausgegeben werden oder die Phase der Hochfrequenzpulse moduliert wird. Nach der Aufnahme liegen die Magnetresonanzdaten der gleichzeitig angeregten Schichten kollabiert als eine einzige Schicht vor. Die Magnetresonanzdaten können in der Nachverarbeitung mittels eines Schichttrennungsalgorithmus, wie beschrieben, getrennt werden. Wird eine Beschleunigung auch in der Schichtebene eingesetzt, beispielsweise eine Unterabtastung, erfolgt danach das Inplane-Unaliasing unter Nutzung eines Unterabtastungsalgorithmus, insbesondere eines Inplane-GRAPPA-Algorithmus, in einem zweiten Schritt. Als Ergebnis der Rekonstruktion wird ein Magnetresonanzbilddatensatz erhalten.

Die Turbospinecho-Sequenz (TSE-Sequenz) ist eine der am meisten eingesetzten Magnetresonanzsequenzen in der Magnetresonanzbildgebung und kann für eine Vielzahl von Untersuchungsbereichen des Patienten verwendet werden. Die TSE-Sequenz besteht aus mehreren Echozügen, wobei in jedem Echozug mehrere k-Raumlinien in Phasenkodierungsrichtung nach einem einzigen Anregungspuls aufgenommen werden. Dies wird erreicht, indem die Spins nach der Aufnahme jeder k-Raumlinie mittels Refokussierungspulsen refokussiert werden. Im Vergleich zur konventionellen Spinecho-Sequenz wird die Aufnahmezeit um die Zahl der refokussierten Echos in einem Echozug, also den sogenannten Turbo-Faktor, beschleunigt.

Die TSE-Sequenz (sowie andere Magnetresonanzsequenzen) können auch für die SMS-Bildgebung eingesetzt werden. Um den Schichttrennungsalgorithmus zu kalibrieren, mithin die kollabierten Multiband-Daten zu "entfalten", werden Referenzdaten eines Einzelband-Referenzscans benötigt, bei dem mithin jede Schicht, die später durch SMS-Bildgebung abgedeckt werden soll, einzeln aufgenommen wird. Bekannte Vorgehensweisen bei der SMS-TSE-Bildgebung nutzen einen TSE- oder Gradientenecho-Referenzscan, der der SMS-Datenaufnahme vorausgeht. Dabei wurde vorgeschlagen, die Referenzdaten sowohl zur Kalibrierung des Schichttrennungsalgorithmus als auch zur Kalibrierung des Unterabtastungsalgorithmus einzusetzen.

Aktuelle TSE-Sequenzen, die eine Unterabtastung in der Schichtebene nutzen, aber keine SMS-Bildgebungstechnik einsetzen, unterstützen die vollständige Abtastung eines Teilbereichs des abgetasteten k-Raums um das k-Raumzentrum während der Aufnahme. Die Magnetresonanzdaten dieses zentralen Teilbereichs werden auf der einen Seite genutzt, um die Unterabtastungsalgorithmen zu kalibrieren, mithin deren Kerne zu ermitteln, auf der anderen Seite aber auch, um die entsprechenden Magnetresonanzdaten unmittelbar für den finalen Magnetresonanzbilddatensatz einzusetzen, entweder, indem eine Mittelung für den zentralen Teilbereich durchgeführt wird oder die (interpolierten) k-Raumlinien des zentralen Teilbereichs vollständig durch die aufgenommenen Magnetresonanzdaten ersetzt werden. Dies ist dem Signal-Rausch-Verhältnis (SNR) zuträglich und reduziert die SNR-Nachteile, die der Unterabtastung in der Schichtebene inhärent sind.

Für SMS-Bildgebungstechniken kann dieses Kalibrierungsverfahren nicht eingesetzt werden, nachdem die Kalibrierung des Schichttrennungsalgorithmus, insbesondere des Schicht-GRAPPA-Algorithmus, nur auf regelmäßig unterabgetasteten k-Räumen möglich ist, auf die der Schichttrennungsalgorithmus dann anzuwenden ist. Daher kann für SMS-Bildgebungstechniken ein reduziertes Signal-zu-Rausch-Verhältnis entstehen. Um diesen SNR-Verlust wenigstens teilweise zu reduzieren, wurde vorgeschlagen, Aufnahmeparameter wie beispielsweise Matrixgröße, Phasenüberabtastung oder Zahl der Mittelungen anzupassen, was äußerst aufwendig ist, nachdem die Optimierung stark abhängig vom verwendeten Magnetresonanzprotokoll ist und ein unmittelbarer Vergleich zwischen Magnetresonanzprotokollen, die SMS-Bildgebung nutzen, und Magnetresonanzprotokollen, die keine SMS-Bildgebung nutzen, erschwert wird.

Der Erfindung liegt daher die Aufgabe zugrunde, eine Möglichkeit zur Verbesserung des Signal-zu-Rausch-Verhältnisses bei simultaner Mehrschichtbildgebung kombiniert mit Unterabtastung in der Schichtebene anzugeben.

Diese Aufgabe wird erfindungsgemäß gelöst durch ein Verfahren, eine Magnetresonanzeinrichtung, ein Computerprogramm und einen elektronisch lesbaren Datenträger gemäß den unabhängigen Ansprüchen. Vorteilhafte Ausgestaltungen ergeben sich aus den Unteransprüchen.

Bei einem Verfahren der eingangs genannten Art ist erfindungsgemäß mithin insbesondere vorgesehen, dass die Magnetresonanzdaten in wenigstens zwei Teilbereichen des abgetasteten k-Raums in der Schichtebene mit einem unterschiedlichen Abtastungsgrad aufgenommen werden und vor der Anwendung des Schichttrennungsalgorithmus in wenigstens zwei jeweils einem Teilbereich zugeordnete Anteile jeweils eines festen Abtastungsgrades aufgeteilt werden, auf die der Schichttrennungsalgorithmus jeweils getrennt angewendet wird, wobei die Anteile zur Ermittlung des Magnetresonanzbilddatensatzes schichtweise rekombiniert werden.

Erfindungsgemäß wird also vorgeschlagen, auch bei Einsatz von SMS-Bildgebung weiterhin zusätzliche k-Raumlinien im eigentlichen Bildgebungsscan zu erfassen, welche zur Verbesserung des Signal-zu-Rausch-Verhältnisses ebenso in den letztendlich rekonstruierten Magnetresonanzdatensatz eingehen. Das bedeutet, es liegen ein vollständig abgetasteter Teilbereich um das k-Raumzentrum und ein unterabgetasteter äußerer Teilbereich des abgetasteten k-Raums vor. Um dennoch eine Schichttrennung durch den einen gleichmäßigen Abtastungsgrad voraussetzenden Schichttrennungsalgorithmus zu ermöglichen, wird vorgeschlagen, die Magnetresonanzdaten geschickt derart aufzuteilen, dass zwei einen regelmäßigen Abtastungsgrad aufweisende Anteile entstehen. Das bedeutet, die Magnetresonanzdaten, die mit wenigstens zwei unterschiedlichen Abtastungsgraden vorliegen, werden in wenigstens zwei Anteile aufgeteilt, die jeweils einen einzigen, für den Anteil konstanten Abtastungsgrad aufweisen. Auf diese Anteile wird dann jeweils der Schichttrennungsalgorithmus angewandt, insbesondere ein Schicht-GRAPPA-Algorithmus, wobei durchaus, wie im Folgenden noch näher dargelegt werden wird, unterschiedliche Schichttrennungskerne eingesetzt werden können. Mit anderen Worten erlaubt es die vorliegende Erfindung insbesondere, die simultane Mehrschichtbildgebungstechnik (SMS-Bildgebungstechnik) mit Aufnahmen zu kombinieren, in denen insbesondere das k-Raumzentrum vollständig abgetastet wird, wobei alle aufgenommenen Magnetresonanzdaten genutzt werden, um ein verbessertes Signal-zu-Rausch-Verhältnis (SNR) zu erhalten. Dies wird erreicht, indem die aufgenommenen "kollabierten" Magnetresonanzdaten im k-Raum geeignet aufgeteilt werden und eine separate Schichttrennung für die jeweiligen Anteile durchgeführt wird. Somit wird ein verbessertes Signal-zu-Rausch-Verhältnis erreicht.

In konkreter Ausgestaltung der Erfindung kann vorgesehen sein, dass als Schichttrennungsalgorithmus ein Schicht-GRAPPA-Algorithmus und/oder als Unterabtastungsalgorithmus ein Inplane-GRAPPA-Algorithmus verwendet wird. Besonders vorteilhaft werden die Magnetresonanzdaten mit einer blipped CAIPI-Technik als Mehrschicht-Bildgebungstechnik aufgenommen. Dabei kann insbesondere insgesamt das im eingangs zitierten Artikel von Setsompop et al. beschriebene Vorgehen eingesetzt werden. Als Magnetresonanzsequenz für die Aufnahme der Magnetresonanzdaten wird bevorzugt eine Turbospinecho-Sequenz (TSE-Sequenz) verwendet, wobei sich das Vorgehen selbstverständlich auch auf andere Magnetresonanzsequenzen anwenden lässt, insbesondere weitere spinecho- oder gradientenechobasierte Sequenzarten.

Eine zweckmäßige Weiterbildung der vorliegenden Erfindung sieht vor, dass die Referenzdaten bei vollständiger Abtastung des k-Raums aufgenommen werden und durch Weglassen eines Teils der Referenzdaten wenigstens ein Schichttrennungskern für den Schichttrennungsalgorithmus ermittelt wird, so dass die künstliche Unterabtastung der Referenzdaten der Unterabtastung des Anteils entspricht, für den der Schichttrennungskern verwendet werden soll. Das bedeutet, es ist durchaus denkbar, für den (getrennt durchzuführenden) Referenzscan eine hohe Abtastung des k-Raums, bevorzugt eine vollständige Abtastung, vorzunehmen, da zur Ermittlung entsprechender, auf unterabgetastete Anteile anzuwendender Schichttrennungskerne eine künstliche Unterabtastung durch Weglassen von Referenzdaten zur Herstellung des entsprechenden Abtastungsgrades erreicht werden kann. Eine derartige Ausgestaltung ist insbesondere dann zweckmäßig, wenn die Referenzdaten auch zur Kalibrierung des Unterabtastungsalgorithmus eingesetzt werden sollen, worauf im Folgenden noch näher eingegangen werden wird, aber in besonders bevorzugter Weise auch dann, wenn mehrere Schichttrennungskerne jeweils für unterschiedliche Abtastungsgrade ermittelt werden sollen, beispielsweise für eine vollständige Abtastung und für eine Unterabtastung gemäß einem bestimmten Unterabtastungsgrad.

Entsprechend sieht eine Ausgestaltung der vorliegenden Erfindung vor, dass bei wenigstens zwei einem unterschiedlichen Abtastungsgrad entsprechenden Anteilen jeweils wenigstens ein entsprechender Schichttrennungskern ermittelt wird. Auf diese Weise können hoch abgetastete Teilbereiche des k-Raums, insbesondere also vollständig abgetastete Teilbereiche des k-Raums, gemeinsam weiterverarbeitet werden, was zu einer besonders guten Verbesserung des Signal-zu-Rausch-Verhältnisses führt.

So kann in einer ersten konkreten Ausgestaltung der vorliegenden Erfindung, die sich auf einen ersten, vollständig abgetasteten Teilbereich des k-Raums, insbesondere um das k-Raumzentrum, und einen zweiten, unterabgetasteten Teilbereich des k-Raums bezieht, vorgesehen sein, dass ein Schichttrennungskern für beide Abtastungsgrade aus den Referenzdaten ermittelt wird, wobei zudem die Aufteilung der Magnetresonanzdaten derart erfolgt, dass ein erster Anteil, der einem Abtastungsgrad vollständiger Abtastung entspricht, alle Magnetresonanzdaten des ersten Teilbereichs umfasst, während für den zweiten Anteil die Magnetresonanzdaten des zweiten Teilbereichs aus Magnetresonanzdaten des ersten Teilbereichs derart ergänzt werden, dass ein durchgängig gleicher Abtastungsgrad vorliegt. Auf diesen ersten Anteil und den zweiten Anteil werden dann die jeweiligen, im Abtastungsgrad entsprechenden Schichttrennungskerne durch den Schichttrennungsalgorithmus angewandt. In diesem ersten konkreten Ausführungsbeispiel der vorliegenden Erfindung ist es dabei besonders vorteilhaft, wenn die getrennten Magnetresonanzdaten des vollständig abgetasteten ersten Teilbereichs auch für die Kalibrierung des Unterabtastungsalgorithmus, insbesondere des In-plane-GRAPPA-Algorithmus, eingesetzt werden. Für die unterabgetasteten schichtgetrennten Magnetresonanzdaten wird dann der Unterabtastungsalgorithmus angewandt, woraufhin die Magnetresonanzdaten beider Anteile wieder rekombiniert werden, entweder durch Ersetzung ergänzter unterabgetasteter Magnetresonanzdaten im ersten Teilbereich durch die vollständig abgetasteten Magnetresonanzdaten oder durch Mittelung beider Magnetresonanzdatensätze. Durch Fourier-Transformationen der rekombinierten Magnetresonanzdaten wird dann der Magnetresonanzbilddatensatz erhalten. In diesem ersten Ausführungsbeispiel hat sich die höchste Verbesserung des Signal-zu-Rausch-Verhältnisses gezeigt.

In einer anderen, vorteilhaften Ausgestaltung der vorliegenden Erfindung kann vorgesehen sein, dass die Aufteilung in Anteile so erfolgt, dass alle Anteile denselben Abtastungsgrad aufweisen. Insbesondere wird dadurch erreicht, dass alle Anteile einen einer Unterabtastung entsprechenden Abtastungsgrad aufweisen, so dass es möglich ist, die Referenzdaten mit dem Abtastungsgrad der Anteile, mithin unterabgetastet, aufzunehmen. Zudem muss nur ein Schichttrennungskern des Schichttrennungsalgorithmus berechnet werden. Insgesamt ergeben sich hierbei die Vorteile, dass der externe Referenzdatensatz unterabgetastet aufgenommen werden kann, wodurch Messzeit eingespart wird, und dass ein reduzierter Rechenaufwand besteht, nachdem nur ein einziger Schichttrennungskern berechnet werden muss.

Ein konkretes, zweites Ausführungsbeispiel der vorliegenden Erfindung sieht dabei vor, dass bei vollständiger Abtastung eines ersten Teilbereichs, insbesondere um das k-Raumzentrum, und Unterabtastung eines den restlichen k-Raum umfassenden zweiten Teilbereichs aus dem ersten Bereich k-Raumlinien einem zweiten, Magnetresonanzdaten des zweiten Teilbereichs umfassenden Anteil unter gedachter Fortsetzung des Abtastungsschemas des zweiten Teilbereichs zugefügt werden und die restlichen Magnetresonanzdaten des ersten Teilbereichs in wenigstens einem ersten Anteil, dessen Unterabtastung der des ersten Anteils entspricht, weiterverarbeitet werden. Insbesondere gilt bei einer Unterabtastung in der Schichtebene um den Faktor 2 als Abtastungsgrad des zweiten Teilbereichs, dass jede zweite Linie des ersten Teilbereichs dem zweiten Anteil zugeordnet wird und die verbleibenden k-Raumlinien des ersten Teilbereichs als ein erster Anteil verwendet werden. Mit anderen Worten wird der vollständig abgetastete, insbesondere zentrale erste Teilbereich in zwei oder mehr Teile aufgeteilt, so dass die vollständig abgetasteten Magnetresonanzdaten mithin auf wenigstens zwei Anteile aufgeteilt werden. Die Zahl der Anteile wird dabei durch den Inplane-Beschleunigungsfaktor bestimmt, beim Faktor 2, wie oben dargelegt, einen einzigen ersten und einen zweiten Anteil, beim Faktor 3 einen zwei zweite Anteile und einen zweiten Anteil usw. Dabei wird das Abtastungsschema des zweiten Teilbereichs, mithin der Abtastungsgrad, jedoch für alle Anteile beibehalten, so dass keiner der Anteile eine vollständig abgetastete Region des k-Raums umfasst und alle Anteile unter Verwendung desselben Schichttrennungskerns im Schichttrennungsalgorithmus schichtgetrennt werden können. Nach dieser Schichttrennung werden alle schichtgetrennten Anteile wieder rekombiniert, wobei wiederum mit besonderem Vorteil der vollständig abgetastete, insbesondere zentrale erste Teilbereich für die Kalibrierung des Unterabtastungsalgorithmus eingesetzt werden kann. Mittels des Unterabtastungsalgorithmus werden dann die fehlenden k-Raumlinien im zweiten Teilbereich ergänzt und der Magnetresonanzdatensatz kann unter Verwendung einer Fourier-Transformation erhalten werden.

Dabei sei darauf hingewiesen, dass es nicht zwangsläufig notwendig ist, die Kalibrierung des Unterabtastungsalgorithmus aus vollständig abgetasteten Magnetresonanzdaten des eigentlichen Bildgebungsscans vorzunehmen. Das bedeutet, es sind auch Ausführungsbeispiele denkbar, in denen wenigstens ein vollständig abgetasteter Teil der Referenzdaten zur Kalibrierung des Unterabtastungsalgorithmus verwendet wird, dennoch jedoch gegenüber einer vollständigen Unterabtastung zusätzliche k-Raumlinien bei der Aufnahme der Magnetresonanzdaten abgetastet werden, die zur Verbesserung des Signal-zu-Rausch-Verhältnisses des resultierenden Magnetresonanzbilddatensatzes eingesetzt werden können, indem sie ebenso schichtgetrennt werden und der Ermittlung des Magnetresonanzbilddatensatzes wieder zugeführt werden, wie oben beschrieben.

Es sei angemerkt, dass es bei vielen Magnetresonanzsequenzen zur Aufnahme der Magnetresonanzdaten bereits Zeitfenster gibt, in denen diese zusätzlichen k-Raumlinien ohne wesentlichen Zeitverlust abgetastet werden können, so dass ein unterschiedliche Abtastungsgrade aufweisender Magnetresonanzdatensatz entsteht, der wie hier beschrieben behandelt werden kann. Das bedeutet aber, eine Verbesserung des Signal-zu-Rauschverhältnisses kann gemäß der vorliegenden Erfindung bereits dann erreicht werden, wenn nur ein relativ kleiner erster Teilbereich vollständig abgetastet wird, der zur Kalibrierung des Unterabtastungsalgorithmus nicht ausreichen würde.

Bevorzugt ist es im Hinblick auf das Signal-zu-Rauschverhältnis jedoch, Magnetresonanzdaten eines Teilbereichs höheren (vollständigen) Abtastungsgrades wenigstens teilweise zur Kalibrierung des Unterabtastungsalgorithmus heranzuziehen, mithin bezüglich des Unterabtastungsalgorithmus wenigstens teilweise eine Kalibrierung integriert im eigentlichen Bildgebungsscan zu erlauben.

Eine besonders zweckmäßige Ausgestaltung kann sich ergeben, wenn sowohl Magnetresonanzdaten eines vollständig abgetasteten Teilbereichs als auch wenigstens ein vollständig abgetasteter Teil der Referenzdaten zur Kalibrierung des Unterabtastungsalgorithmus verwendet werden. Das bedeutet, Referenzdaten für den Unterabtastungsalgorithmus können sich sowohl aus dem externen Referenzscan als auch aus vollständig abgetasteten Teilbereichen des Bildgebungsscans ergeben, so dass eine weitere Verbesserung der Stabilität und der Robustheit gegenüber Bewegung bezüglich der Kalibrierung des Unterabtastungsalgorithmus entsteht.

Neben dem Verfahren betrifft die vorliegende Erfindung auch eine Magnetresonanzeinrichtung, aufweisend eine zur Ausführung des erfindungsgemäßen Verfahrens ausgebildete Steuereinrichtung. Sämtliche Ausführungen bezüglich des erfindungsgemäßen Verfahrens lassen sich analog auf die erfindungsgemäße Magnetresonanzeinrichtung übertragen, mit welcher mithin ebenso die bereits genannten Vorteile erhalten werden können. Die Steuereinrichtung, welche beispielsweise wenigstens einen Prozessor und/oder wenigstens ein Speichermittel aufweisen kann, kann hierzu beispielsweise zu einer Sequenzeinheit zur Aufnahme der Referenzdaten und der Magnetresonanzdaten sowie einer Kalibrierungseinheit zur Kalibrierung des Schichttrennungsalgorithmus und des Unterabtastungsalgorithmus auch eine Aufteilungseinheit zur Aufteilung der Magnetresonanzdaten in die Anteile aufweisen. Eine Rekonstruktionseinheit kann sowohl die Anwendung des Schichttrennungsalgorithmus als auch die Anwendung des Unterabtastungsalgorithmus und die Ermittlung des eigentlichen Magnetresonanzbilddatensatzes übernehmen, wobei sie zusätzlich zur Rekombination der getrennt schichtgetrennten Anteile der Magnetresonanzdaten ausgebildet ist.

Ein erfindungsgemäßes Computerprogramm ist beispielsweise direkt in einen Speicher einer Steuereinrichtung einer Magnetresonanzeinrichtung ladbar und weist Programmmittel auf, um die Schritte des erfindungsgemäßen Verfahrens auszuführen, wenn das Computerprogramm in der Steuereinrichtung der Magnetresonanzeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen und derart ausgestaltet sind, dass sie bei Verwendung des Datenträgers in einer Steuereinrichtung einer Magnetresonanzeinrichtung ein erfindungsgemäßes Verfahren durchführen. Bei dem erfindungsgemäßen Datenträger kann es sich insbesondere um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Skizze zur Erläuterung eines ersten Ausführungsbeispiels des erfindungsgemäßen Verfahrens,
- Fig. 2: eine Skizze zur Erläuterung eines zweiten Ausführungsbeispiels des erfindungsgemäßen Verfahrens,
- Fig. 3: eine Skizze zur Erläuterung eines dritten Ausführungsbeispiels des erfindungsgemäßen Verfahrens, und
- Fig. 4: eine erfindungsgemäße Magnetresonanzeinrichtung.

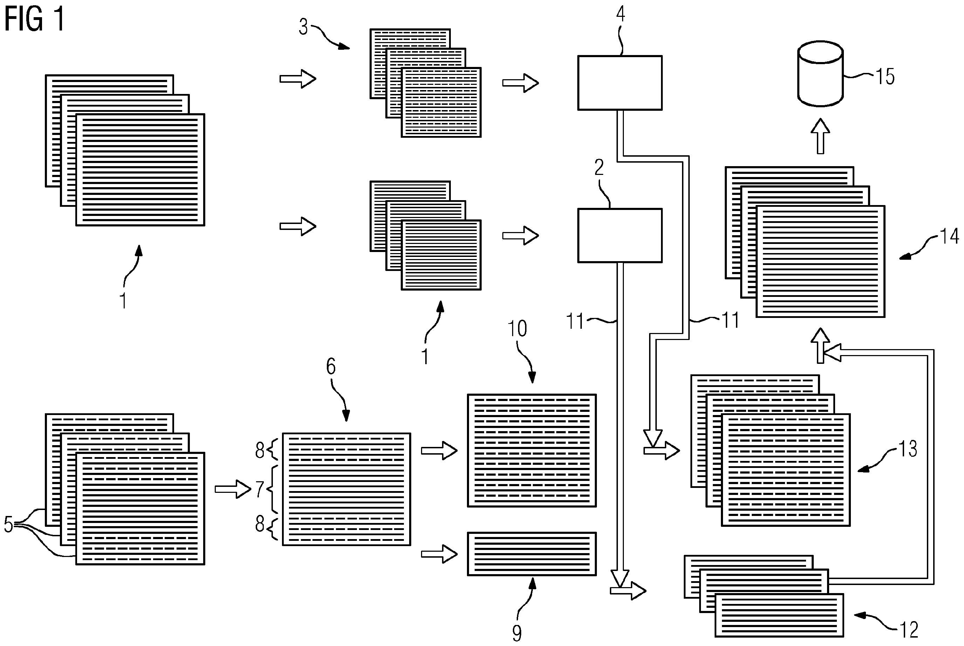

Fig. 1 erläutert ein erstes Ausführungsbeispiel des erfindungsgemäßen Verfahrens näher. Dabei werden zunächst in einem ersten Schritt Referenzdaten 1 für alle später aufzunehmenden Schichten, in diesem Fall in vollständiger Abtastung des abzutastenden k-Raums, aufgenommen. Hierfür kann beispielsweise eine Gradientenecho-Sequenz eingesetzt werden. Die vollständigen Referenzdaten 1, also bei vollständiger Abtastung, werden zur Ermittlung von ersten Schichttrennungskernen 2 eines Schichttrennungsalgorithmus, hier eines Schicht-GRAPPA-Algorithmus, verwendet. Daneben wird jedoch durch Weglassen einzelner k-Raumlinien der Referenzdaten 1 ein künstlich unterabgetasteter Referenzdatensatz 3 erzeugt, wobei dies vorliegend für einen Abtastungsgrad gezeigt ist, der in der Schichtebene einen Faktor 2 der Unterabtastung beschreibt, so dass jede zweite k-Raumlinie gestrichelt, also nicht abgetastet, gezeigt ist. Aus den künstlich unterabgetasteten Referenzdaten 3 werden zweite Schichttrennungskerne 4 für den Schicht-GRAPPA-Algorithmus ermittelt, so dass letztlich Schichttrennungskerne 2, 4 für unterschiedliche Abtastungsgrade, nämlich vollständige Abtastung und Unterabtastung, hier um den Faktor 2, vorliegen.

Bei der späteren Aufnahme von Magnetresonanzdaten werden in dem Bildgebungsscan mehrere Schichten 5 mit einer simultanen Mehrschicht-Bildgebungstechnik gleichzeitig vermessen, so dass so genannte "kollabierte" Magnetresonanzdaten 6 für alle Schichten 5 gemeinsam entstehen. Neben der SMS-Bildgebungstechnik wurden die Magnetresonanzdaten 6 innerhalb der Schichtebene in einem ersten Teilbereich 7 vollständig abgetastet, in einem zweiten Teilbereich 8 jedoch mit dem Abtastungsgrad der Referenzdaten 3 unterabgetastet, wie wiederum durch gestrichelte k-Raumlinien als ausgelassene k-Raumlinien gekennzeichnet ist. Ersichtlich erstreckt sich der erste Teilbereich 7 um das k-Raumzentrum. Dabei sei angemerkt, dass der erste Teilbereich 7 deutlich kleiner sein kann; die Größen in Fig. 1 (sowie den weiteren Figuren) sind lediglich zur deutlichen Erkennbarkeit gewählt.

In einem folgenden Schritt werden die Magnetresonanzdaten 6 nun in einen ersten Anteil 9 und einen zweiten Anteil 10 aufgeteilt. Der erste Anteil 9 entspricht den Magnetresonanzdaten 6 des vollständig abgetasteten ersten Teilbereichs 7, während der zweite Anteil 10 den Magnetresonanzdaten 6 des zweiten Teilbereichs 8 ergänzt um Magnetresonanzdaten 6 des ersten Teilbereichs 7 derart, dass sich das Abtastschema, wie dargestellt, fortsetzt, entspricht. Der erste Anteil 9 weist also einen einer vollständigen Abtastung entsprechenden Abtastgrad auf, während der zweite Anteil 10 einen der Unterabtastung des zweiten Teilbereichs 8 entsprechenden Abtastungsgrad aufweist.

In einem folgenden Schritt wird der Schichttrennungsalgorithmus getrennt auf beide Anteile 9, 10 angewandt, wobei für den ersten Anteil 9 die ersten Schichttrennungskerne 2 und für den zweiten Anteil 10 die zweiten Schichttrennungskerne 4 verwendet werden, wie durch die Pfeile 11 angedeutet ist.

Als Ergebnis erhält man schichtgetrennte Anteile 12, 13, wobei der schichtgetrennte Anteil 12 zur Kalibrierung eines Unterabtastungsalgorithmus, hier eines Inplane-GRAPPA-Algorithmus verwendet wird. Der Inplane-GRAPPA-Algorithmus wird zumindest auf die dem zweiten Teilbereich 8 zugehörigen schichtgetrennten Magnetresonanzdaten 6 des schichtgetrennten Anteils 13 angewendet, um fehlende Magnetresonanzdaten zu ergänzen. Dann werden die Anteile 12 und 13 zu schichtgetrennten, ergänzten, den gesamten abgetasteten k-Raum vollständig abdeckenden Magnetresonanzdaten 14 rekombiniert, wobei für den ersten Teilbereich 7 entweder unmittelbar der schichtgetrennte erste Anteil 12 verwendet werden kann oder aber eine Mittelung mit den ergänzten schichtgetrennten Magnetresonanzdaten des schichtgetrennten zweiten Anteils 13 im ersten Teilbereich 7 erfolgen kann.

Aus den schichtgetrennten, ergänzten Magnetresonanzdaten 14 kann dann, wie bekannt, ein Magnetresonanzbilddatensatz 15 rekonstruiert werden.

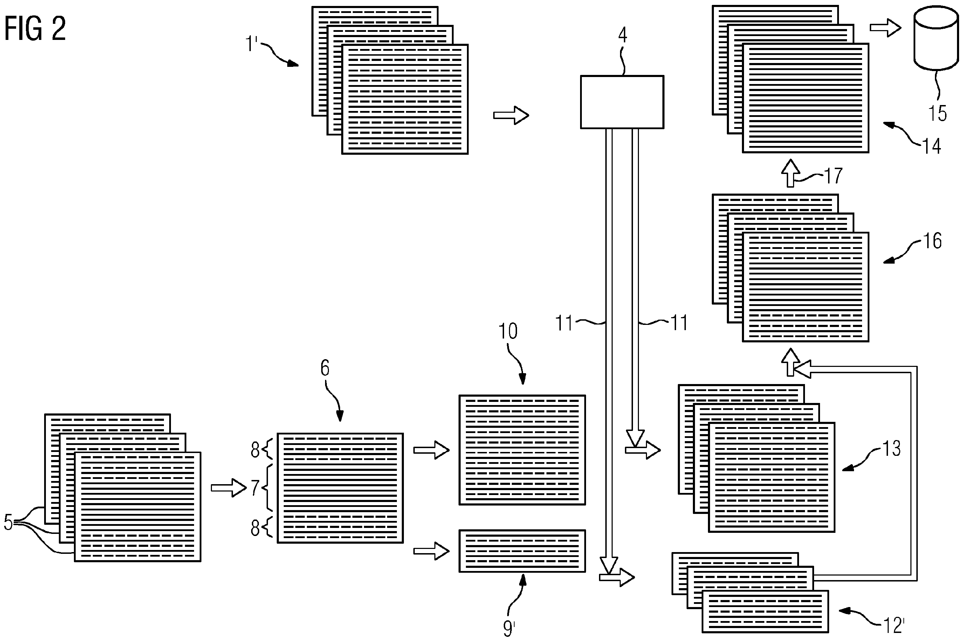

Fig. 2 stellt ein gegenüber dem ersten Ausführungsbeispiel der Fig. 1 modifiziertes zweites Ausführungsbeispiel dar, in dem die Referenzdaten 1' mit dem Abtastungsgrad des zweiten Teilbereichs 8 unterabgetastet aufgenommen werden können und lediglich die Schichttrennungskerne 4 für diesen Abtastungsgrad berechnet werden müssen. Dies wird ermöglicht, indem die Magnetresonanzdaten 6 in einen ersten Anteil 9' und einen zweiten Anteil 10 derart aufgeteilt werden, dass die Anteile 9', 10 jeweils den Abtastungsgrad der Unterabtastung im zweiten Teilbereich 8 aufweisen. Hierzu wird der zweite Anteil 10 wie zu Fig. 1 beschrieben gebildet, wobei der erste Anteil 9' nun jedoch nur noch die verbleibenden, nicht im zweiten Anteil 10 hinzugefügten k-Raumlinien des ersten Teilbereichs 7 umfasst. Dabei sei angemerkt, dass dies für den hier verwendeten Unterabtastungsfaktor 2 gilt; bei höheren Unterabtastungsfaktoren in der Schichtebene werden mehrere erste Anteile 9' erzeugt, so dass für alle generierten Anteile 9', 10 derselbe Abtastungsgrad vorliegt, der dem Abtastungsgrad der Referenzdaten 1' entspricht, für den die Schichttrennungskerne 4 ermittelt wurden. Das bedeutet, die Schichttrennungskerne 4 können gemäß der Pfeile 11 für alle Anteile 9', 10 herangezogen werden.

Die entstehenden schichtgetrennten Anteile 12', 13 werden zu schichtgetrennten vorläufigen Magnetresonanzdaten 16 zusammengeführt, wobei hier wiederum die schichtgetrennten vorläufigen Magnetresonanzdaten 16 des ersten Teilbereichs 7 für jede der Schichten 5 zur Kalibrierung des Unterabtastungsalgorithmus herangezogen werden kann. Nach dessen Anwendung, vgl. Pfeil 17, liegen wiederum die schichtgetrennten Magnetresonanzdaten 14 vor, aus denen der Magnetresonanzbilddatensatz 15 rekonstruiert wird.

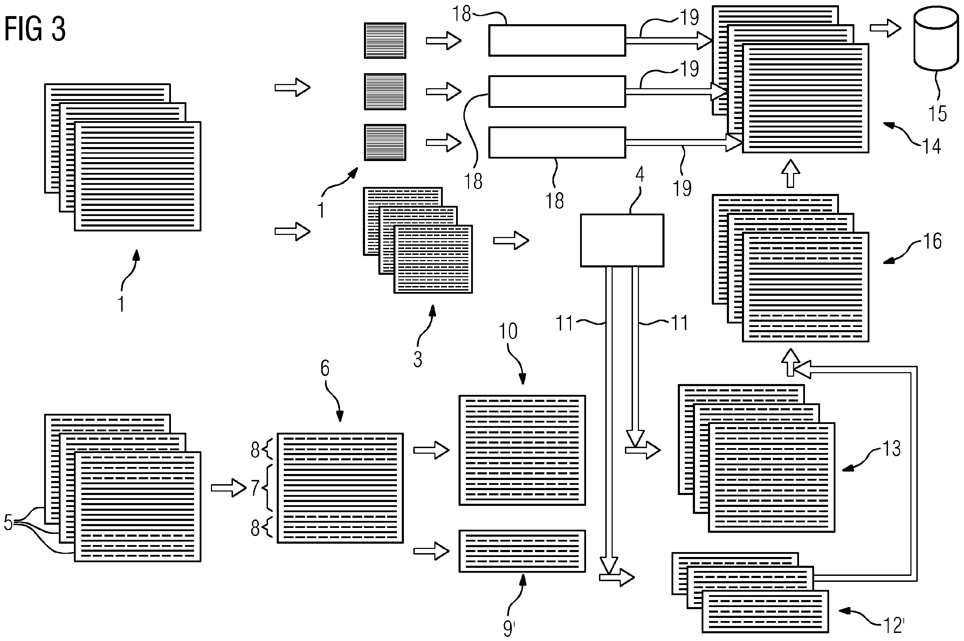

Fig. 3 zeigt nun ein drittes Ausführungsbeispiel des erfindungsgemäßen Verfahrens, das sich gegenüber dem zweiten Ausführungsbeispiel gemäß Fig. 2 darin unterscheidet, dass die Referenzdaten 1 wiederum vollständig abgetastet aufgenommen werden, weiterhin jedoch nur ein Satz von Schichttrennungskernen 4 für künstlich unterabgetastete Referenzdaten 3 errechnet wird. Die Referenzdaten 1 für die einzelnen Schichten 5 werden jedoch zusätzlich zur Ermittlung von Unterabtastungskernen 18 für die einzelnen Schichten 5 herangezogen, welche dann gemäß der Pfeile 19 verwendet werden, um aus den schichtgetrennten vorläufigen Magnetresonanzdaten 16 die schichtgetrennten Magnetresonanzdaten 14 zur Rekonstruktion des Magnetresonanzbilddatensatzes 15 zu erhalten.

Das dritte Ausführungsbeispiel lässt sich insbesondere dann vorteilhaft einsetzen, wenn der vollständig abgetastete erste Teilbereich 7 zu klein für eine verlässliche Kalibrierung des Unterabtastungsalgorithmus, hier des Inplane-GRAPPA-Algorithmus, ist.

Beim ersten und beim dritten Ausführungsbeispiel können vorteilhaft sowohl Referenzdaten 1 als auch schichtgetrennte Messergebnisse des ersten Teilbereichs 7 zur Kalibrierung des Unterabtastungsalgorithmus herangezogen werden, was die Stabilität und Bewegungsrobustheit für die Inplane-GRAPPA-Kalibrierung erhöht.

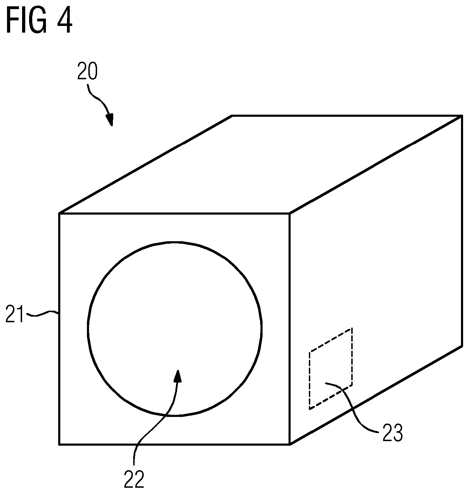

Fig. 4 zeigt schließlich eine Prinzipskizze einer erfindungsgemäßen Magnetresonanzeinrichtung 20, die, wie grundsätzlich bekannt, eine Hauptmagneteinheit 21 aufweist, in der eine Patientenaufnahme 22 definiert ist, in die ein Patient mittels einer hier nicht näher gezeigten Patientenliege einfahrbar ist. Die Patientenaufnahme 22 umgebend können eine Hochfrequenzspulenanordnung und eine Gradientenspulenanordnung der Magnetresonanzeinrichtung 20 vorgesehen sein.

Der Betrieb der Magnetresonanzeinrichtung 20 wird durch eine Steuereinrichtung 23 gesteuert, die zur Durchführung des erfindungsgemäßen Verfahrens ausgebildet ist. Hierzu kann die Steuereinrichtung 23 neben einer Sequenzeinheit und einer Rekonstruktionseinheit insbesondere auch eine Aufteilungseinheit zur Aufteilung in die Anteile 9 bzw. 9' und 10 aufweisen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Aufnahme eines Magnetresonanzbilddatensatzes eines Untersuchungsbereichs eines Patienten mit einer Magnetresonanzeinrichtung, wobei zur Aufnahme von Magnetresonanzdaten (6, 14, 16) eine Mehrschicht-Bildgebungstechnik bei gleichzeitig zumindest teilweiser Unterabtastung in der Schichtebene angewandt wird, wobei Magnetresonanzdaten (6, 14, 16) aus mehreren angeregten Schichten (5) gleichzeitig ausgelesen und durch einen Schichttrennungsalgorithmus, der mittels in einem getrennten Referenzscan aufgenommenen Referenzdaten (1, 1', 3) kalibriert ist, den gleichzeitig ausgelesenen Schichten (5) zugeordnet werden, wonach ein die Unterabtastung in der Schichtebene kompensierender Unterabtastungsalgorithmus auf die unterabgetasteten Magnetresonanzdaten (16) der einzelnen Schichten (5) angewandt wird, **dadurch gekennzeichnet, dass** die Magnetresonanzdaten (6) in wenigstens zwei Teilbereichen (7, 8) des abgetasteten k-Raums in der Schichtebene mit einem unterschiedlichen Abtastungsgrad aufgenommen werden, mit einem vollständig abgetasteten Teilbereich (7) um das k-Raumzentrum und einem unterabgetasteten äusseren Teilbereich (8) des abgetasteten k-Raums, und vor der Anwendung des Schichttrennungsalgorithmus in wenigstens zwei jeweils einem Teilbereich (7, 8) zugeordnete Anteile (9, 9', 10) jeweils eines festen Abtastungsgrades aufgespalten werden, auf die der Schichttrennungsalgorithmus jeweils getrennt mit einem entsprechenden Schichttrennungskern angewendet wird, wobei die Anteile (9, 9', 10) zur Ermittlung des Magnetresonanzbilddatensatzes (15) schichtweise rekombiniert werden.

2. Verfahren nach Anspruch 1, **dadurch gekennzeichnet, dass** die Referenzdaten (1, 1', 3) bei vollständiger Abtastung des k-Raums aufgenommen werden und durch Weglassen eines Teils der Referenzdaten (1, 1', 3) wenigstens ein Schichttrennungskern (2, 4) für den Schichttrennungsalgorithmus ermittelt wird, so dass die künstliche Unterabtastung der Referenzdaten (1, 1', 3) der Unterabtastung des Anteils (9, 9', 10) entspricht, für den der Schichttrennungskern (2, 4) verwendet werden soll.

3. Verfahren nach Anspruch 2, **dadurch gekennzeichnet, dass** bei wenigstens zwei einem unterschiedlichen Abtastungsgrad entsprechenden Anteilen (9, 9', 10) jeweils wenigstens ein entsprechender Schichttrennungskern (2, 4) ermittelt wird.

4. Verfahren nach Anspruch 1 oder 2, **dadurch gekennzeichnet, dass** die Aufteilung in Anteile (9, 9', 10) so erfolgt, dass alle Anteile (9', 10) denselben Abtastungsgrad aufweisen.

5. Verfahren nach Anspruch 4, **dadurch gekennzeichnet, dass** die Referenzdaten (1') mit dem Abtastungsgrad der Anteile (9', 10) aufgenommen werden.

6. Verfahren nach Anspruch 4 oder 5, **dadurch gekennzeichnet, dass** bei vollständiger Abtastung eines ersten Teilbereichs (7), insbesondere um das k-Raumzentrum, und Unterabtastung eines den restlichen k-Raum umfassenden zweiten Teilbereichs (8) aus dem ersten Teilbereich (7) k-Raumlinien einem zweiten, Magnetresonanzdaten (6) des zweiten Teilbereichs (8) umfassenden Anteil (10) unter gedachter Fortsetzung des Abtastungsschemas des zweiten Teilbereichs (8) zugefügt werden und die restlichen Magnetresonanzdaten (6) des ersten Teilbereichs (7) in wenigstens einem ersten Anteil (9'), dessen Unterabtastung der des zweiten Anteils (10) entspricht, weiterverarbeitet werden.

7. Verfahren nach einem der vorangehenden Ansprüche, **dadurch gekennzeichnet, dass** Magnetresonanzdaten (6, 14, 16) eines Teilbereichs (7) höheren Abtastungsgrades und/oder wenigstens ein vollständig abgetasteter Teil der Referenzdaten (1) zur Kalibrierung des Unterabtastungsalgorithmus verwendet werden.

8. Verfahren nach einem der vorangehenden Ansprüche, **dadurch gekennzeichnet, dass** als Schichttrennungsalgorithmus ein Schicht-GRAPPA-Algorithmus und/oder als Unterabtastungsalgorithmus ein Inplane-GRAPPA-Algorithmus verwendet wird und/oder die Magnetresonanzdaten (6) mit einer Blipped-CAIPI-Technik als Mehrschicht-Bildgebungstechnik aufgenommen werden und/oder das als Magnetresonanzsequenz für die Aufnahme der Magnetresonanzdaten (6) eine TSE-Sequenz verwendet wird.

9. Magnetresonanzeinrichtung (20), aufweisend eine zur Ausführung eines Verfahrens nach einem der vorangehenden Ansprüche ausgebildete Steuereinrichtung (23).

10. Computerprogramm, welches die Schritte eines Verfahrens nach einem der Ansprüche 1 bis 8 durchführt, wenn es auf einer Steuereinrichtung (23) einer Magnetresonanzeinrichtung (20) ausgeführt wird.

11. Elektronisch lesbarer Datenträger, auf dem ein Computerprogramm nach Anspruch 10 gespeichert ist.

## Claims

1. Method for recording a magnetic resonance image data set of an investigation region of a patient with a magnetic resonance device, wherein for the recording of magnetic resonance data (6, 14, 16) a multislice imaging technique is applied with simultaneous at least partial undersampling in the slice plane, wherein magnetic resonance data (6, 14, 16) is read out from a plurality of excited slices (5) simultaneously and, by means of a slice separation algorithm which is calibrated by means of reference data (1, 1', 3) recorded in a separate reference scan, is allocated to the simultaneously read-out slices (5), after which an undersampling algorithm compensating for the undersampling in the slice plane is applied to the undersampled magnetic resonance data (16) of the individual slices (5), **characterised in that** the magnetic resonance data (6) is recorded with a different degree of sampling in at least two subregions (7, 8) of the sampled k-space in the slice plane, with a completed sampled subregion (7) about the k-space centre and an undersampled outer subregion (8) of the sampled k-space, and before the use of the slice separation algorithm is split into at least two portions (9, 9', 10) that are each associated with one subregion (7, 8), each of one fixed degree of sampling, to each of which the slice separation algorithm is applied separately with a corresponding slice separation core, wherein the portions (9, 9', 10) are recombined slice by slice to determine the magnetic resonance image data set (15).

2. Method according to claim 1, **characterised in that** the reference data (1, 1', 3) is recorded with complete sampling of the k-space and by leaving out a part of the reference data (1, 1', 3), at least one slice separation kernel (2, 4) for the slice separation algorithm is determined, so that the artificial undersampling of the reference data (1, 1', 3) corresponds to the undersampling of the portion (9, 9', 10) for which the slice separation kernel (2, 4) is to be used.

3. Method according to claim 2, **characterised in that** with at least two portions (9, 9', 10), each corresponding to a different degree of sampling, at least one corresponding slice separation kernel (2, 4) is determined in each case.

4. Method according to claim 1 or 2, **characterised in that** the distribution into portions (9, 9', 10) takes place such that all the portions (9', 10) have the same degree of sampling.

5. Method according to claim 4, **characterised in that** the reference data (1') is recorded with the degree of sampling of the portions (9', 10).

6. Method according to claim 4 or 5, **characterised in that** on complete sampling of a first subregion (7), in particular about the k-space centre and undersampling of a second subregion (8) comprising the remaining k-space, from the first subregion (7) k-space lines are added to a second portion (10) comprising magnetic resonance data (6) of the second subregion (8) with imagined continuation of the sampling pattern of the second subregion (8), and the remaining magnetic resonance data (6) of the first subregion (7) in at least one first portion (9'), the undersampling of which corresponds to that of the second portion (10), is further processed.

7. Method according to one of the preceding claims, **characterised in that** magnetic resonance data (6, 14, 16) of a subregion (7) of a higher degree of sampling and/or at least one completely sampled portion of the reference data (1) is used for calibrating the undersampling algorithm.

8. Method according to one of the preceding claims, **characterised in that** as the slice separation algorithm, a slice GRAPPA algorithm and/or as the undersampling algorithm, an in-plane GRAPPA algorithm is used and/or the magnetic resonance data (6) is recorded with a blipped CAIPI technique as the multislice imaging technique and/or **in that** as the magnetic resonance sequence for the recording of the magnetic resonance data (6), a TSE sequence is used.

9. Magnetic resonance device (20), having a control device (23) configured for carrying out a method according to one of the preceding claims.

10. Computer program which carries out the steps of a method according to one of claims 1 to 8 when it is executed on a control device (23) of a magnetic resonance device (20).

11. Electronically readable data carrier on which a computer program according to claim 10 is stored.

## Revendications

1. Procédé d'enregistrement d'un ensemble de données d'image de résonnance magnétique d'une partie à examiner d'un patient par un dispositif de résonnance magnétique, dans lequel, pour l'enregistrement des données (6, 14, 16) de résonnance magnétique, on applique une technique d'imagerie à plusieurs couches avec sous-échantillonnage simultané au moins partiel dans le plan de couche, dans lequel on lit simultanément des données (6, 14, 16) de résonnance magnétique de plusieurs couches (5) excitées et on les associe aux couches (5) lues simultanément par un algorithme de séparation de couches, qui est étalonné au moyen de données (1, 1', 3) de référence enregistrées dans un scan de référence séparés, ensuite on applique un algorithme de sous-échantillonnage compensant le sous-échantillonnage dans le plan de couches aux données (16) de résonnance magnétique sous-échantillonnées des diverses couches (5),
**caractérisé en ce que** l'on enregistre les données (6) de résonnance magnétique dans au moins deux régions (7, 8) partielles de l'espace k échantillonné dans le plan de couches à un degré d'échantillonnage différent, avec une région (7) partielle échantillonnée complètement autour du centre de l'espace k et une région (8) partielle extérieure sous-échantillonnée de l'espace k échantillonné, et avant l'application de l'algorithme de séparation de couches, on sépare au moins deux proportions (9, 9', 10), associées respectivement à une région (7, 8) partielle, respectivement d'un degré d'échantillonnage fixe, proportions sur lesquelles l'algorithme de séparation de couches est appliqué respectivement séparément avec un noyau de séparation de couche correspondant, les proportions (9, 9', 10) étant recombinées couche par couche pour la détermination de l'ensemble (15) de données d'image de résonnance magnétique.

2. Procédé suivant la revendication 1, **caractérisé en ce que** l'on enregistre les données (1, 1', 3) de référence à un échantillonnage complet de l'espace k et, en délaissant une partie des données (1, 1', 3) de référence, on détermine au moins un noyau (2, 4) de séparation de couche pour l'algorithme de séparation de couches, de manière à ce que le sous-échantillonnage artificiel des données (1, 1', 3) de référence correspondent au sous-échantillonnage de la proportion (9, 9', 10) pour laquelle le noyau (2, 4) de séparation de couche doit être utilisé.

3. Procédé suivant la revendication 2, **caractérisé en ce que**, pour au moins deux proportions (9, 9', 10) correspondant à un degré d'échantillonnage différent, on détermine respectivement au moins un noyau (2, 4) de séparation de couche correspondant.

4. Procédé suivant la revendication 1 ou 2, **caractérisé en ce qu'**on effectue la séparation en proportions (9, 9', 10) de manière à ce que toutes les proportions (9', 10) aient le même degré d'échantillonnage.

5. Procédé suivant la revendication 4, **caractérisé en ce que** l'on enregistre les données (1') de référence au degré d'échantillonnage des proportions (9', 10).

6. Procédé suivant la revendication 4 ou 5, **caractérisé en ce que**, pour un échantillonnage complet d'une première région (7) partielle, notamment autour du centre de l'espace k et un sous - échantillonnage d'une deuxième région (8) partielle entourant l'espace k restant, on ajoute des lignes de l'espace k provenant de la première région (7) partielle à une deuxième proportion (10), comprenant des données (6) de résonnance magnétique de la deuxième région (8) partielle, en continuation imaginaire du schéma d'échantillonnage de la deuxième région (8) partielle et on retraite les données (6) de résonnance magnétique restantes de la première région (7) partielle en au moins une première proportion (9'), dont le sous-échantillonnage correspond à celui de la deuxième proportion (10).

7. Procédé suivant l'une des revendications précédentes, **caractérisé en ce que** l'on utilise des données (6, 14, 16) de résonnance magnétique d'une région (7) partielle de degré d'échantillonnage plus grand et/ou au moins une partie échantillonnée complètement des données (1) de référence pour l'étalonnage de l'algorithme de sous-échantillonnage.

8. Procédé suivant l'une des revendications précédentes, **caractérisé en ce que** l'on utilise comme algorithme de séparation de couche, un algorithme GRAPPA de couche et/ou comme algorithme de sous-échantillonnage un algorithme GRAPPA-Inplane et/ou on enregistre les données (6) de résonnance magnétique par une technique Blipped-CAIPI comme technique d'imagerie à plusieurs couches et/ou **en ce que** l'on utilise une séquence TSE comme séquence de résonnance magnétique pour l'enregistrement des données (6) de résonnance magnétique.

9. Dispositif (20) de résonnance magnétique comportant un dispositif (23) de commande constitué pour l'exécution d'un procédé suivant l'une des revendications précédentes.

10. Programme d'ordinateur, qui effectue un procédé suivant l'une des revendications 1 à 8, lorsqu'il est réalisé sur un dispositif (23) de commande d'un dispositif (20) de résonnance magnétique.

11. Support de données déchiffrable électroniquement, sur lequel est mémorisé un programme d'ordinateur suivant la revendication 10.
